# EUROPÄISCHE PATENTSCHRIFT

(11) **EP 2 061 880 B1**
(45) Veröffentlichungstag und Bekanntmachung des Hinweises auf die Patenterteilung: **09.12.2009**
(21) Anmeldenummer: 07803080.6
(22) Anmeldetag: 30.08.2007
(51) Int. Cl.: C12N 9/02, C07C 5/09, C12P 7/00

(54) **VERFAHREN ZUR ENZYMATISCHEN REDUKTION VON ALKINDERIVATEN**
METHOD FOR THE ENZYMATIC REDUCTION OF ALKYNE DERIVATES
PROCEDE DE REDUCTION ENZYMATIQUE DE DERIVES D'ALCYNES

(30) Priorität: 01.09.2006 EP 06120008
(43) Veröffentlichungstag der Anmeldung: 27.05.2009
(73) Patentinhaber: BASF SE, 67056 Ludwigshafen (DE)
(72) Erfinder: STÜRMER, Rainer, 67127 Rödersheim-Gronau (DE); HAUER, Bernhard, 67136 Fussgönheim (DE); ROSCHE, Bettina, Randwick, New South Wales 2031 (AU); MUELLER, André, Bondi Beach, Sydney, New South Wales 2026 (AU)
(86) Internationale Anmeldenummer: PCT/EP2007/059071
(87) Internationale Veröffentlichungsnummer: WO 2008/025831

(56) Entgegenhaltungen:
- TAKESHITA M ET AL: "Asymmetric biotransformation of phenyl-C4 derivatives in rat liver (S- 9) and baker's yeast" JOURNAL OF MOLECULAR CATALYSIS - B ENZYMATIC 14 SEP 1998 NETHERLANDS, Bd. 5, Nr. 1-4, 14. September 1998 (1998-09-14), Seiten 245-248, XP002428124 ISSN: 1381-1177
- KITAMURA SHIGEYUKI ET AL: "Reductive metabolism of an alpha,beta-ketoalkyne, 4-phenyl-3-butyn-2-one, by rat liver preparations" DRUG METABOLISM AND DISPOSITION, Bd. 30, Nr. 4, April 2002 (2002-04), Seiten 414-420, XP002428125 ISSN: 0090-9556
- SAITO K ET AL: "The cloning and expression of a gene encoding Old Yellow Enzyme from Saccharomyces carlsbergensis." THE JOURNAL OF BIOLOGICAL CHEMISTRY 5 NOV 1991, Bd. 266, Nr. 31, 5. November 1991 (1991-11-05), Seiten 20720-20724, XP002428126 ISSN: 0021-9258
- STOTT KELVIN ET AL: "Old Yellow Enzyme: The discovery of multiple isozymes and a family of related proteins" JOURNAL OF BIOLOGICAL CHEMISTRY, AMERICAN SOCIETY OF BIOLOCHEMICAL BIOLOGISTS, BIRMINGHAM,, US, Bd. 268, Nr. 9, 25. März 1993 (1993-03-25), Seiten 6097-6106, XP002252405 ISSN: 0021-9258
- NIINO YUKO S ET AL: "A new Old Yellow Enzyme of Saccharomyces cerevisiae" JOURNAL OF BIOLOGICAL CHEMISTRY, AMERICAN SOCIETY OF BIOLOCHEMICAL BIOLOGISTS, BIRMINGHAM,, US, Bd. 270, Nr. 5, 3. Februar 1995 (1995-02-03), Seiten 1983-1991, XP002252406 ISSN: 0021-9258

## Beschreibung

Die Erfindung betrifft ein Verfahren zur enzymatischen Reduktion von Alkinderivaten.

### Stand der Technik

Bisher sind keine Verfahren zur enzymatischen Reduktion von Alkinderivaten bekannt, die kein ATP benötigen.

Takeshita, M. et al. beschreiben in Journal of Molecular Catalysis B: Enzymatic 5 (1998) 245-248 Versuche zur asymmetrischen Biotransformation von Phenyl-C₄ Derivaten mit der Rattenleberfraktion S-9 und mit Bäckerhefe. Danach wird 4-Phenyl-3-butin-2-on von der Rattenleberfraktion im Vergleich zur Kontrolle nicht signifikant in das korrespondierende Buten-2-on überführt. Auch die Versuche mit ganzen Hefezellen lieferten eine insignifikante Menge (3% Ausbeute) an Buten-2-on. Dagegen wurde als Hauptkomponente das korrespondierende S-Butan-2-ol (31%), das korrespondierende Butan-2-on (8%) und das korrespondierende S-Butin-2-ol (5%) gebildet. Diesen Biotransformationen konnten keine bestimmten Enyzmaktivitiäten zugeordnet werden, da die Versuche mit ganzen Hefezellen durchgeführt wurden. Außerdem erfolgten die Versuche zwangsläufig in Gegenwart zellulärer Cofaktoren, wie z.B. ATP.

Es bestand die Aufgabe, ein Verfahren zur enzymatischen Reduktion von Alkinderivaten bereitzustellen, das kein ATP benötigt.

### Kurzfassung der Erfindung

Obige Aufgabe wurde gelöst durch Verwendung der Reduktasen OYE 1, 2 und 3 und funktionaler Äquivalente davon zur Reduktion von Alkinderivaten der allgemeinen Formel (1).

### Figurenbeschreibung

In den beiliegenden Figuren zeigt
Figur 1 die Biotransformation von 20 mM 4-Phenyl-3-butin-2-on mit OYE 1-3 in Gegenwart von NADPH (links) oder NADH (rechts), EDTA und Isopropanol bei pH 6.8 und 30°C nach 1 h. Die Enzyme wurden in *E*. *coli* TG10+ überexprimiert, TG10+ selbst diente als Kontrolle;

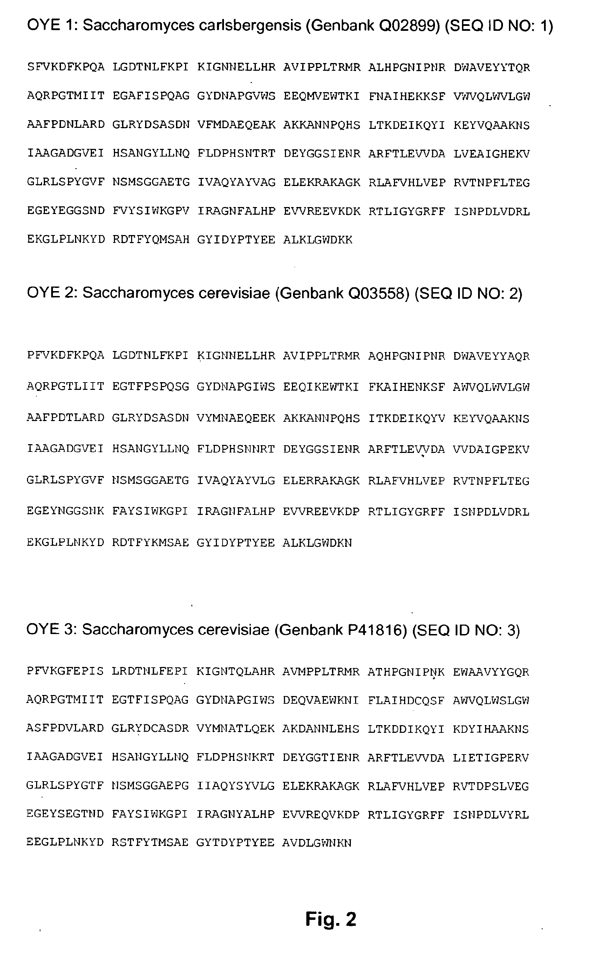

Figur 2 die Aminosäuresequenzen der verwendeten Enzyme OYE 1, 2 und 3;
Figur 3 die Plasmidkarte von pAgro4;
Figur 4 die Plasmidkarte von pHSG575';
Figur 5 die Plasmidkarte von pDHE1658 OYE1;
Figur 6 die Plasmidkarte von pDHE1658 OYE2; und
Figur 7 die Plasmidkarte von pDHE1658 OYE3.

### Detaillierte Beschreibung der Erfindung

Die vorliegende Erfindung betrifft ein Verfahren zur enzymatischen Herstellung von Alkenon-Derivaten der allgemeinen Formel (2) aus α,β- ungesättigten Alkinon-Derivaten der allgemeinen Formel (1) 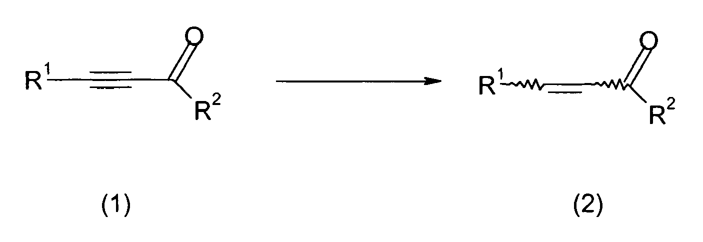 worin
R¹ für H, C₁-C₆- Alkyl, C₂-C₆- Alkenyl, oder einen gegebenenfalls substituierten carbo- oder hetrocyclischen aromatischen oder nicht aromatischen Ring steht, und
R² für H, C₁-C₆- Alkyl oder C₂-C₆-Alkenyl steht,
durch Reduktion einer Verbindung der Formel (1) in Gegenwart einer Reduktase
(i) umfassend wenigstens eine der Polypeptidsequenzen SEQ ID NO:1, 2, 3, 5, 7 oder 9 oder
(ii) mit einer funktional äquivalenten Polypeptidsequenz, die mindestens 80% Sequenzidentität mit SEQ ID NO:1, 2, 3, 5, 7 oder 9 aufweist.

Vorzugsweise wird erfindungsgemäß insbesondere die E-Konfiguration von Verbindungen der Formel 2 bereitgestellt, 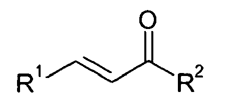 worin R¹ und R² die oben angegebenen Bedeutungen besitzen. Insbesondere liegen die Verbindungen der Formel 2 zu mehr als 50%, insbesondere mehr als 60, 70 oder 80 % vorzugsweise aber zu mehr als 90%, wie z.B. 95 bis 99%, insbesondere zu etwa 100% in der E-Konfiguration vor.

Grundsätzlich ist das erfindungsgemäße Verfahren sowohl mit gereinigten oder angereicherten Enzym selbst als auch mit Mikroorganismen, welche dieses Enzym natürlich oder rekombinant exprimieren, mit davon abgeleiteten Zellhomogenaten oder, falls der Mikroorganismus das Enzym in die Umgebung ausscheidet, mit Kulturübersand durchführbar. Insbesondere umfasst eine "enzymatische Umsetzung" im Sinne der Erfindung aber eine Umsetzung in einer im Wesentlichen ATP-freien Umgebung, d.h. bevorzugt eine Umsetzung mit zellfreien Enzympräparaten, wie reinen oder angereicherten Enzymen oder geeigneten Proteinfraktionen, welche keine niedermolekularen zellulären Bestandteile, wie insbesondere ATP, mehr enthalten.

Werden keine anderen Angaben gemacht, so bedeutet:
- C₁-C₆-Alkyl insbesondere Methyl, Ethyl, Propyl, Butyl, Pentyl oder Hexyl, sowie die entsprechenden ein- oder mehrfach verzweigte Analoga, wie i-Propyl, i-Butyl, sec.-Butyl, tert.-Butyl, i-Pentyl oder Neopentyl; wobei insbesondere die genannten C₁-C₄-Alkylreste bevorzugt sind;
- C₂-C₆-Alkenyl insbesondere die einfach ungesättigten Analoga der oben genannten Alkylreste mit 2 bis 6 Kohlenstoffatomen, wobei insbesondere die entsprechenden C₂-C₄-Alkenylreste bevorzugt sind.
- Carbo- und heterocyclische aromatische oder nichtaromatische Ringe insbesondere gegebenenfalls kondensierte Ringe mit 3 bis 12 Kohlenstoffatomen und gegebenenfalls 1 bis 4 Heteroatomen, wie N, S und O, insbesondere N oder O. Als Beispiele können genannt werden Cyclopropyl, Cyclobutyl, Cyclopentyl, Cyclohexyl, Cycloheptyl, die ein- oder mehrfach ungesättigten Analoga davon, wie Cyclobutenyl, Cyclopentenyl, Cyclohexenyl, Cycloheptenyl, Cyclohexadienyl, Cycloheptadienyl; Phenyl und Naphthyl; sowie 5- bis 7-gliedrige gesättigte oder ungesättigte heterocyclische Reste mit 1 bis 4 Heteroatomen, die ausgewählt sind unter O, N und S, wobei der Heterocyclus gegebenenfalls mit einem weitern Heterocyclus oder Carbocyclus kondensiert sein kann. Insbesondere sind zu nennen heterocyclische Reste abgeleitet von Pyrrolidin, Tetrahydrofuran, Piperidin, Morpholin, Pyrrol, Furan, Thiophen, Pyrazol, Imidazol, Oxazol, Thiazol, Pyridin, Pyran, Pyrimidin, Pyridazin, Pyrazin, Cumaron, Indol und Chinolin. Gegebenenfalls können die cyclischen Reste, aber auch die oben genannten Alkyl-und Alkenylreste ein- oder mehrfach, wie z. B. 1-, 2- oder 3-fach, substituiert sein. Als Beispiel für geeignete Substituierte sind zu nennen: Halogen, insbesondere F, Cl, Br; - OH, -SH, -NO₂ -NH₃, -SO₃H, C₁-C₄-Alkyl und C₂-C₄-Alkenyl, C₁-C₄ -Alkoxy; und Hydroxy-C₁-C₄-Alkyl; wobei die Alkyl- und Alkenyl-Reste wie oben definiert sind und die Alkoxy-Reste von den oben definierten korrespondierenden Alkylresten abgeleitet sind.

Das erfindungsgemäße Verfahren kann insbesondere mit Alkinen der allgemeinen Formel (1) durchgeführt werden, in denen R¹ für C₁-C₄- Alkyl in verzweigter und unverzweigter Form, C₂-C₆ Alkenyl in verzweigter und unverzweigter Form oder gegebenenfalls substituiertes Phenyl steht.

Besonders geeignete Substrate für das erfindungsgemäße Verfahren sind solche Alkine der allgemeinen Formel (1), bei denen R¹ für gegebenenfalls substituiertes Phenyl steht und R² für CH₃ steht.

Die erfindungsgemäß verwendeten Reduktasen reduzieren gelegentlich teilweise neben der Dreifachbindung in α,β-Position zur Carbonylfunktion auch die Carbonylfunktion selbst, wodurch dann der entsprechende Alkohol gebildet wird. Ebenso können die Alkene teilweise zum korrespondierenden Alkan weitereduziert werden.

Für das erfindungsgemäße Verfahren geeignete Reduktasen sind all diejenigen Enzyme, die in der Lage sind, 4-Phenyl-3-butin-2-on in einer NAD(P)H abhängigen, und vorzugsweise ATP-unabhängigen, Reaktion zu E-4-Phenyl-3-buten-2-on zu reduzieren. Diese Reaktion wird im Folgenden auch Modellreaktion genannt.

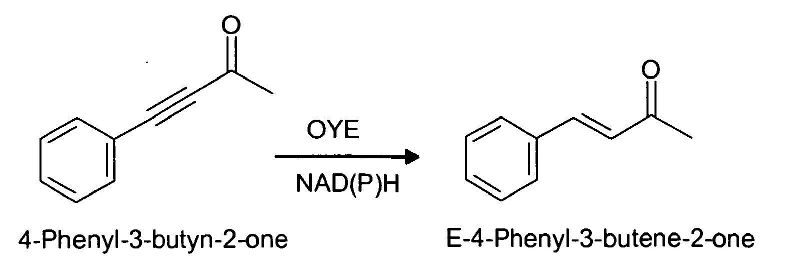

Weiterhin besitzen die für das erfindungsgemäße Verfahren geeigneten Reduktasen (welche zuweilen auch als Enoat-Reduktasen bezeichnet werden) eine Polypeptidsequenz gemäss SEQ ID NO:1, 2, 3, 5, 7 oder 9 oder eine Polypeptidsequenz, die mindestens 80%, wie z.B. mindestens 90%, oder mindestens 95% und insbesondere mindestens 97%, 98% oder 99% Sequenzidentität mit SEQ ID NO: 1, 2, 3, 5, 7 oder 9 aufweist.

Ein Polypeptid mit der SEQ ID NO:1 ist bekannt unter der Bezeichnung OYE1 aus Saccharomyces carlsbergensis (Genbank Q02899).

Ein Polypeptid mit der SEQ ID NO:2 wird kodiert vom OYE2 Gen aus Bäckerhefe (Saccharomyces cerevisiae Genlocus YHR179W) (Genbank Q03558).

Ein Polypeptid mit der SEQ ID NO:3 wird kodiert vom OYE3 Gen aus Bäckerhefe (Saccharomyces cerevisiae Genlocus YPL171C) (Genbank P 41816).

Die Sequenzen gemäß SEQ ID NO: 5, 7 und 9 entsprechen SEQ ID NO: 1, 2 und 3 und unterscheiden sich davon lediglich durch einen zusätzlichen N-terminalen Methioninrest.

Die Ermittlung der Sequenzidentität soll für die hier beschriebenen Zwecke durch das Computerprogramm "GAP" der Genetics Computer Group (GCG) der University of Wisconsin erfolgen, wobei die Version 10.3 unter Verwendung der von GCG empfohlenen Standardparameter zum Einsatz kommen soll.

Solche Reduktasen können ausgehend von SEQ ID NO: 1, 2, 3, 5, 7 oder 9 durch dem Fachmann bekannte gezielte oder randomisierte Mutageneseverfahren erhalten werden. Alternativ kann jedoch auch in Mikroorganismen , bevorzugt in solchen der Gattungen *Alishewanella, Alterococcus, Aquamonas, Aranicola, Arsenophonus, Azotivirga, Brenneria, Buchnera (aphid P-endosymbionts), Budvicia, Buttiauxella, Candidatus Phlomobacter, Cedecea, Citrobacter, Dickeya, Edwardsiella, Enterobacter, Erwinia, Escherichia, Ewingella, Grimontella, Hafnia, Klebsiella, Kluyvera, Leclercia, Leminorella, Moellerella, Morganella, Obesumbacterium, Pantoea, Pectobacterium, Photorhabdus, Plesiomonas, Pragia, Proteus, Providencia, Rahnella, Raoultella, Salmonella, Samsonia, Serratia, Shigella, Sodalis, Tatumella, Trabulsiella, Wigglesworthia, Xenorhabdus, Yersinia* oder *Yokenella,* nach Reduktasen gesucht werden, die die o.g. Modellreaktion katalysieren und deren Aminosäuresequenz die geforderte Sequenzidentität zu SEQ ID NO: 1, 2, 3, 5, 7 oder 9 bereits aufweist oder durch Mutageneseverfahren erhalten wird.

Die Reduktase kann in gereinigter oder teilweise gereinigter Form oder auch in Form des Mikroorganismus selbst verwendet werden. Verfahren zur Gewinnung und Aufreinigung von Dehydrogenasen aus Mikroorganismen sind dem Fachmann hinreichend bekannt.

Bevorzugt erfolgt die die enantioselektive Reduktion mit der Reduktase in Gegenwart eines geeigneten Cofaktors (auch als Cosubstrat bezeichnet). Als Cofaktoren für die Reduktion des Ketons dient üblicherweise NADH und/oder NADPH. Daneben können Reduktasen als zelluläre Systeme eingesetzt werden, die inherent Cofaktor enthalten, oder alternative Redoxmediatoren zugesetzt werden (A. Schmidt, F. Hollmann und B. Bühler "Oxidation of Alcohols" in K. Drauz und H. Waldmann, Enzyme Catalysis in Organic Synthesis 2002, Vol.III, 991-1032, Wiley-VCH,Weinheim).

Bevorzugt erfolgt die enantioselektive Reduktion mit der Reduktase außerdem in Gegenwart eines geeigneten Reduktionsmittels, welches den im Verlauf der Reduktion oxidierten Cofaktor regeneriert. Beispiele für geeignete Reduktionsmittels sind Zucker, insbesondere Hexosen, wie Glucose, Mannose, Fructose, und/oder oxidierbare Alkohole, insbesondere Ethanol, Propanol oder Isopropanol, sowie Formiat, Phosphit oder molekularer Wasserstoff. Zur Oxidation des Reduktionsmittels und damit verbunden zur Regeneration des Coenzyms kann eine zweite Dehydrogenase, wie z.B. Glucose-dehydrogenase bei Verwendung von Glucose als Reduktionsmittel oder Formiat-Dehydrogenase bei der Verwendung von Formiat als Reduktionsmittel, zugesetzt werden. Diese kann als freies oder immobilisiertes Enzym oder in Form von freien oder immobilisierten Zellen eingesetzt werden. Ihre Herstellung kann sowohl separat als auch durch Coexpression in einem (rekombinanten) Reduktase-Stamm erfolgen.

Eine bevorzugte Ausführungsform des beanspruchten Verfahrens ist die Regenerierung der Cofaktoren durch ein enzymatisches System, bei dem eine zweite Dehydrogenase, besonders bevorzugt eine Glucosedehydrogenase, verwendet wird.

Weiterhin kann es zweckmäßig sein, weitere die Reduktion fördernde Zusätze, wie z. B. Metallsalze oder Chelatbildner, wie. z. B. EDTA, zu zusetzten.

Die erfindungsgemäß verwendeten Reduktasen können frei oder immobilisiert eingesetzt werden. Unter einem immobilisierten Enzym versteht man ein Enzym, das an einen inerten Träger fixiert ist. Geeignete Trägermaterialien sowie die darauf immobilisierten Enzyme sind aus der EP-A-1149849, EP-A-1 069 183 und der DE-OS 10019377 sowie aus den darin zitierten Literaturstellen bekannt. Auf die Offenbarung dieser Schriften wird diesbezüglich in vollem Umfang Bezug genommen. Zu den geeigneten Trägermaterialien gehören beispielsweise Tone, Tonmineralien, wie Kaolinit, Diatomeenerde, Perlit, Siliciumdioxid, Aluminiumoxid, Natriumcarbonat, Calciumcarbonat, Cellulosepulver, Anionenaustauschermaterialien, synthetische Polymere, wie Polystyrol, Acrylharze, Phenolformaldehydharze, Polyurethane und Polyolefine, wie Polyethylen und Polypropylen. Die Trägermaterialien werden zur Herstellung der geträgerten Enzyme üblicherweise in einer feinteiligen, partikelförmigen Form eingesetzt, wobei poröse Formen bevorzugt sind. Die Partikelgröße des Trägermaterials beträgt üblicherweise nicht mehr als 5 mm, insbesondere nicht mehr als 2 mm (Sieblinie). Analog kann bei Einsatz der Dehydrogenase als Ganzzell-Katalysator eine freie oder immobiliserte Form gewählt werden. Trägermaterialien sind z.B. Ca-Alginat, und Carrageenan. Enzyme wie auch Zellen können auch direkt mit Glutaraldehyd vernetzt werden (Crosslinking zu CLEAs). Entsprechende und weitere Immobilisierungsverfahren sind beispielsweise in J. Lalonde und A. Margolin "Immobilization of Enzymes" in K. Drauz und H. Waldmann, Enzyme Catalysis in Organic Synthesis 2002, Vol.III, 991-1032, Wiley-VCH, Weinheim beschrieben.

Die Umsetzung kann in wässrigen oder nichtwässrigen Reaktionsmedien oder in 2-Phasensystemen oder (Mikro-)Emulsionen erfolgen. Bei den wässrigen Reaktionsmedien handelt es sich vorzugsweise um gepufferte Lösungen, die in der Regel einen pH-Wert von 4 bis 8, vorzugsweise von 5 bis 8, aufweisen. Das wässrige Lösungsmittel kann neben Wasser außerdem wenigstens einen Alkohol, z.B. Ethanol oder Isopropanol oder Dimethylsulfoxid enthalten.

Unter nicht-wässrigen Reaktionsmedien werden Reaktionsmedien verstanden, die weniger als 1 Gew.-%, vorzugsweise weniger als 0,5 Gew.-% Wasser, bezogen auf das Gesamtgewicht des flüssigen Reaktionsmediums, enthalten. Insbesondere kann die Umsetzung in einem organischen Lösungsmittel durchgeführt werden.

Geeignete organische Lösungsmittel sind beispielsweise aliphatische Kohlenwasserstoffe, vorzugsweise mit 5 bis 8 Kohlenstoffatomen, wie Pentan, Cyclopentan, Hexan, Cyclohexan, Heptan, Octan oder Cyclooctan, halogenierte aliphatische Kohlenwasserstoffe, vorzugsweise mit einem oder zwei Kohlenstoffatomen, wie Dichlormethan, Chloroform, Tetrachlorkohlenstoff, Dichlorethan oder Tetrachlorethan, aromatische Kohlenwasserstoffe, wie Benzol, Toluol, die Xylole, Chlorbenzol oder Dichlorbenzol, aliphatische acyclische und cyclische Ether oder Alkohole, vorzugsweise mit 4 bis 8 Kohlenstoffatomen, wie Ethanol, Isopropanol, Diethylether, Methyl-tert-butylether, Ethyl-tert-butylether, Dipropylether, Diisopropylether, Dibutylether, Tetrahydrofuran oder Ester wie Ethylacetat oder n-Butylacetat oder Ketone wie Methylisobutylketon oder Dioxan oder Gemische davon. Besonders bevorzugt werden die vorgenannten Ether, insbesondere Tetrahydrofuran, verwendet.

Beispielsweise kann die Reduktion mit der Reduktase in einem wässrig-organischen Reaktionsmedium, wie z. B. Wasser/Isopropanol, in beliebigem Mischungsverhältnis wie z.B. 1:99 bis 99:1 oder 10: 90 bis 90:10, oder einem wässrigen Reaktionsmedium durchgeführt werden.

Das Substrat (1) wird vorzugsweise in einer Konzentration von 0,1 g/l bis 500 g/l, besonders bevorzugt von 1 g/l bis 50 g/l in die enzymatische Reduktion eingesetzt und kann kontinuierlich oder diskontinuierlich nachgeführt werden.

Die enzymatische Reduktion erfolgt in der Regel bei einer Reaktionstemperatur unterhalb der Desaktivierungstemperatur der eingesetzten Reduktase und oberhalb von -10 °C. Besonders bevorzugt liegt sie im Bereich von 0 bis 100 °C, insbesondere von 15 bis 60 °C und speziell von 20 bis 40 °C, z.B. bei etwa 30 °C.

Zur Durchführung kann man beispielsweise das Substrat (1) mit der Reduktase, dem Lösungsmittel und gegebenenfalls den Coenzymen, gegebenenfalls einer zweiten Dehydrogenase zur Regenerierung des Coenzyms und/oder weiteren Reduktionsmitteln vorlegen und das Gemisch durchmischen, z. B. durch Rühren oder Schütteln. Es ist aber auch möglich, die Reduktase in einem Reaktor, beispielsweise in einer Säule, zu immobilisieren, und durch den Reaktor eine das Substrat und gegebenenfalls Coenzyme und/oder Cosubstrate enthaltende Mischung zu leiten. Hierzu kann man die Mischung im Kreislauf durch den Reaktor leiten bis der gewünschte Umsatz erreicht ist.

Dabei wird die Dreifachbindung in α,β-Position zur Carbonylfunktion zur Doppelbindung reduziert; gelegentlich erfolgt auch eine Reduktion der Carbonylfunktion selbst zur Alkoholfunktion. In der Regel wird man die Reduktion bis zu einem Umsatz von wenigstens 70 %, besonders bevorzugt von wenigstens 85 % und insbesondere von wenigstens 95%, bezogen auf das in der Mischung enthaltene Substrat führen. Das Fortschreiten der Reaktion, d. h. die sequentielle Reduktion der Doppelbindung kann dabei durch übliche Methoden wie Gaschromatographie oder Hochdruckflüssigkeitschromatographie verfolgt werden.

"Funktionale Äquivalente" oder Analoga der konkret offenbarten Enzyme sind im Rahmen der vorliegenden Erfindung davon verschiedene Polypeptide, welche weiterhin die gewünschte biologische Aktivität, wie z.B. Substratspezifität, besitzen. So versteht man beispielsweise unter "funktionalen Äquivalenten" Enzyme, die die Modellreaktion katalysieren und die mindestens 20 %, bevorzugt 50 %, besonders bevorzugt 75 %, ganz besonders bevorzugt 90 % der Aktivität eines Enzyms, umfassend eine der unter SEQ ID NO:1, 2 oder 3 aufgeführten Aminosäuresequenzen, aufweisen. Funktionale Äquivalente sind außerdem vorzugsweise zwischen pH 4 bis 10 stabil und besitzen vorteilhaft ein pH-Optimum zwischen pH 5 und 8 sowie ein Temperaturoptimum im Bereich von 20°C bis 80°C.

Unter "funktionalen Äquivalenten" versteht man erfindungsgemäß insbesondere auch Mutanten, welche in wenigstens einer Sequenzposition der oben genannten Aminosäuresequenzen eine andere als die konkret genannte Aminosäure aufweisen aber trotzdem eine der oben genannten biologischen Aktivitäten besitzen. "Funktionale Äquivalente" umfassen somit die durch eine oder mehrere Aminosäure-Additionen, - Substitutionen, -Deletionen und/oder -Inversionen erhältlichen Mutanten, wobei die genannten Veränderungen in jeglicher Sequenzposition auftreten können, solange sie zu einer Mutante mit dem erfindungsgemäßen Eigenschaftsprofil führen. Funktionale Äquivalenz ist insbesondere auch dann gegeben, wenn die Reaktivitätsmuster zwischen Mutante und unverändertem Polypeptid qualitativ übereinstimmen, d.h. beispielsweise gleiche Substrate mit unterschiedlicher Geschwindigkeit umgesetzt werden.

Beispiele für geeignete Aminosäuresubstitutionen sind folgender Tabelle zu entnehmen:

| Ursprünglicher Rest | Beispiele der Substitution |
|---|---|
| Ala | Ser |
| Arg | Lys |
| Asn | Gln; His |
| Asp | Glu |
| Cys | Ser |
| Gln | Asn |
| Glu | Asp |
| Gly | Pro |
| His | Asn ; Gln |
| Ile | Leu; Val |
| Leu | Ile; Val |
| Lys | Arg ; Gln ; Glu |
| Met | Leu ; Ile |
| Phe | Met ; Leu ; Tyr |
| Ser | Thr |
| Thr | Ser |
| Trp | Tyr |
| Tyr | Trp ; Phe |
| Val | Ile; Leu |

"Funktionale Äquivalente" im obigen Sinne sind auch "Präkursoren" der beschriebenen Polypeptide sowie "funktionale Derivate" .

"Präkursoren" sind dabei natürliche oder synthetische Vorstufen der Polypeptide mit oder ohne der gewünschten biologischen Aktiviät.

"Funktionale Derivate" erfindungsgemäßer Polypeptide können an funktionellen Aminosäure-Seitengruppen oder an deren N- oder C-terminalen Ende mit Hilfe bekannter Techniken ebenfalls hergestellt werden. Derartige Derivate umfassen beispielsweise aliphatische Ester von Carbonsäuregruppen, Amide von Carbonsäuregruppen, erhältlich durch Umsetzung mit Ammoniak oder mit einem primären oder sekundären Amin; N-Acylderivate freier Aminogruppen, hergestellt durch Umsetzung mit Acylgruppen; oder O-Acylderivate freier Hydroxygruppen, hergestellt durch Umsetzung mit Acylgruppen.

Im Falle einer möglichen Proteinglykosylierung umfassen erfindungsgemäße "funktionale Äquivalente" Proteine des oben bezeichneten Typs in deglykosylierter bzw. glykosylierter Form sowie durch Veränderung des Glykosylierungsmusters erhältliche abgewandelte Formen.

"Funktionale Äquivalente" umfassen natürlich auch Polypeptide welche aus anderen Organismen zugänglich sind, sowie natürlich vorkommende Varianten. Beispielsweise lassen sich durch Sequenzvergleich Bereiche homologer Sequenzregionen festlegen und in Anlehnung an die konkreten Vorgaben der Erfindung äquivalente Enzyme ermitteln.

"Funktionale Äquivalente" umfassen ebenfalls Fragmente, vorzugsweise einzelne Domänen oder Sequenzmotive, der erfindungsgemäßen Polypeptide, welche z.B. die gewünschte biologische Funktion aufweisen.

"Funktionale Äquivalente" sind außerdem Fusionsproteine, welche eine der oben genannten Polypeptidsequenzen oder davon abgeleitete funktionale Äquivalente und wenigstens eine weitere, davon funktionell verschiedene, heterologe Sequenz in funktioneller N- oder C-terminaler Verknüpfung (d.h. ohne gegenseitigen wesentliche funktionelle Beeinträchtigung der Fusionsproteinteile) aufweisen. Nichtlimitierende Beispiele für derartige heterologe Sequenzen sind z.B. Signalpeptide oder Enzyme.

Homologe des erfindungsgemäßen Proteine können durch Screening kombinatorischer Banken von Mutanten, wie z.B. Verkürzungsmutanten, identifiziert werden. Beispielsweise kann eine variegierte Bank von Protein-Varianten durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtlicher Sequenzen in einem Gemisch, die den gewünschten Satz an potentiellen Proteinsequenzen kodieren. Verfahren zur Synthese degenerierter Oligonukleotide sind dem Fachmann bekannt (z.B. Narang, S.A. (1983) Tetrahedron 39:3; Itakura et al. (1984) Annu. Rev. Biochem. 53:323; Itakura et al., (1984) Science 198:1056; Ike et al. (1983) Nucleic Acids Res. 11:477).

Im Stand der Technik sind mehrere Techniken zum Screening von Genprodukten kombinatorischer Banken, die durch Punktmutationen oder Verkürzung hergestellt worden sind, und zum Screening von cDNA-Banken auf Genprodukte mit einer ausgewählten Eigenschaft bekannt. Diese Techniken lassen sich an das schnelle Screening der Genbanken anpassen, die durch kombinatorische Mutagenese erfindungsgemäßer Homologer erzeugt worden sind. Die am häufigsten verwendeten Techniken zum Screening großer Genbanken, die einer Analyse mit hohem Durchsatz unterliegen, umfassen das Klonieren der Genbank in replizierbare Expressionsvektoren, Transformieren der geeigneten Zellen mit der resultierenden Vektorenbank und Exprimieren der kombinatorischen Gene unter Bedingungen, unter denen der Nachweis der gewünschten Aktivität die Isolation des Vektors, der das Gen kodiert, dessen Produkt nachgewiesen wurde, erleichtert. Recursive-Ensemble-Mutagenese (REM), eine Technik, die die Häufigkeit funktioneller Mutanten in den Banken vergrößert, kann in Kombination mit den Screeningtests verwendet werden, um Homologe zu identifizieren (Arkin und Yourvan (1992) PNAS 89:7811-7815; Delgrave et al. (1993) Protein Engineering 6(3):327-331).

Gégenstand der Erfindung sind weiterhin Nukleinsäuresequenzen (einzel- und doppelsträngige DNA- und RNA-Sequenzen, wie z.B. cDNA und mRNA), die für ein Enzym mit erfindungsgemäßer Reduktase -Aktivität kodieren. Bevorzugt sind Nukleinsäuresequenzen, welche z.B. für Aminosäuresequenzen gemäß SEQ ID NO:1, 2 oder 3 oder charakteristische Teilsequenzen davon kodieren.

Alle hierin erwähnten Nukleinsäuresequenzen sind in an sich bekannter Weise durch chemische Synthese aus den Nukleotidbausteinen, wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoramiditmethode (Voet, Voet, 2. Auflage, Wiley Press New York, Seiten 896-897) erfolgen. Die Anlagerung synthetischer Oligonukleotide und Auffüllen von Lücken mit Hilfe des Klenow-Fragmentes der DNA-Polymerase und Ligationsreaktionen sowie allgemeine Klonierungsverfahren werden in Sambrook et al. (1989), Molecular Cloning: A laboratory manual, Cold Spring Harbor Laboratory Press, beschrieben.

Weitere Ausgestaltungen zur Durchführung des erfindungsgemäßen enzymatischen Reduktionsverfahrens:

Die erfindungsgemäß eingesetzten Reduktasen können im erfindungsgemäßen Verfahren als freies oder immobilisiertes Enzym verwendet werden.

Der pH-Wert im erfindungsgemäßen Verfahren wird vorteilhaft zwischen pH 4 und 12, bevorzugt zwischen pH 4,5 und 9, besonders bevorzugt zwischen pH 5 und 8 gehalten.

Für das erfindungsgemäße Verfahren können wachsende Zellen verwendet werden, die für die Reduktase kodierenden Nukleinsäuren, Nukleinsäurekonstrukte oder Vektoren enthalten. Auch ruhende oder aufgeschlossene Zellen können verwendet werden. Unter aufgeschlossenen Zellen sind beispielsweise Zellen zu verstehen, die über eine Behandlung mit beispielsweise Lösungsmitteln durchlässig gemacht worden sind, oder Zellen die über eine Enzymbehandlung, über eine mechanische Behandlung (z.B. French Press oder Ultraschall) oder über eine sonstige Methode aufgebrochen wurden. Die so erhaltenen Rohextrakte sind für das erfindungsgemäße Verfahren vorteilhaft geeignet. Auch gereinigte oder angereinigte Enzyme können für das Verfahren verwendet werden. Ebenfalls geeignet sind immobilisierte Mikroorganismen oder Enzyme, die vorteilhaft in der Reaktion Anwendung finden können.

Das erfindungsgemäße Verfahren kann batchweise, semi-batchweise oder kontinuierlich betrieben werden.

Die Durchführung des Verfahrens kann vorteilhafterweise in Bioreaktoren erfolgen, wie z.B. beschrieben in Biotechnology, Band 3, 2. Auflage, Rehm et al Hrsg., (1993) insbesondere Kapitel II.

Die nachfolgenden Beispiele sollen die Erfindung veranschaulichen, ohne sie jedoch einzuschränken. Hierbei wird auf beiliegende Abbildungen Bezug genommen. Experimenteller Teil

### Beispiel 1: Herstellung der Reduktase exprimierenden E. coli TG10⁺ Transformanten und der korrespondierenden Transformationskonstrukte

Werden keine anderen Angaben gemacht, so können die im Rahmen der vorliegenden Erfindung durchgeführten Klonierungsschritte, wie z.B. Restriktionsspaltungen, Agarose Gelelektrophorese, Reinigung von DNA-Fragmenten, Transfer von Nukleinsäuren auf Nitrozellulose und Nylonmembranen, Verknüpfen von DNA-Fragmenten, Transformation von Mikroorganismen, Anzucht von Mikrorganismen, Vermehrung von Phagen und Sequenzanalyse rekombinanter DNA, wie bei Sambrook et al. (1989) a.a.O. beschrieben durchgeführt werden.

Der rekombinante Escherichia-coli-Stamm TG10⁺(OYE) wurde wie folgt konstruiert:

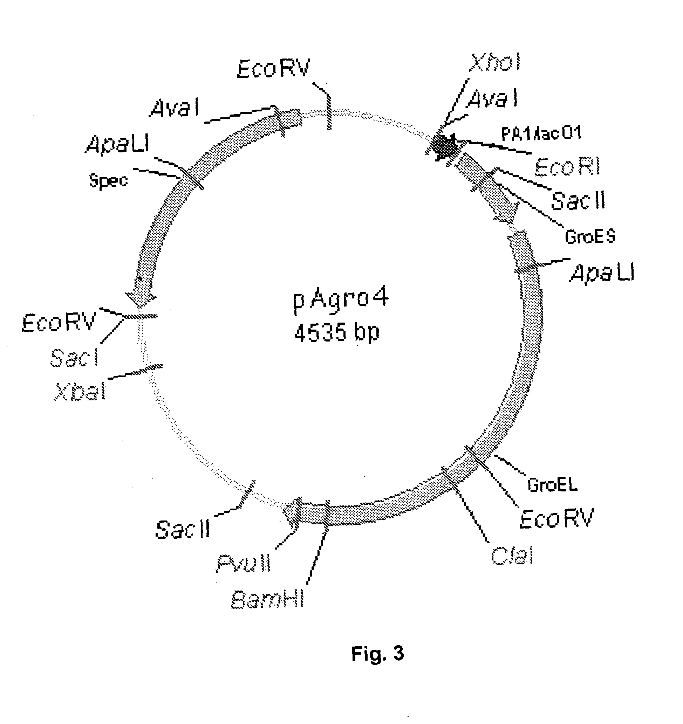

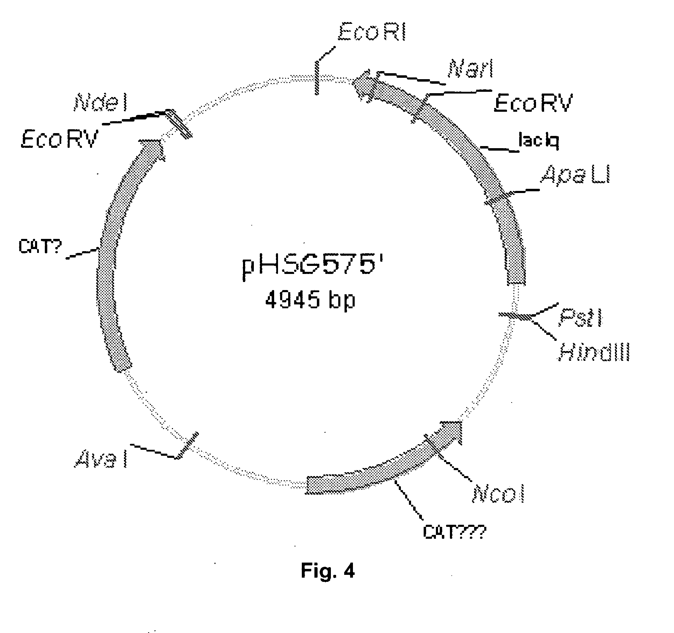

E.coli TG10 ist von E. coli TG1 (Stratagene) abgeleitet. TG10 ist ein tetracyclinresistenter und Rhamnose auxotropher Stamm. TG10⁺ ist von TG10 durch Einführung der folgenden Plasmide abgeleitet:
a) pAgro4 (Chaperon + Streptomycinresistenz) (vgl. Figur 3) und
b) pHSG575 (Chaperon + Chloramphenicolresistenz) (vgl. Figur 4).
pAgro4 ist ein Abkömmling der pZ-Vektoren mit groELS-Chaperoningenen aus E. coli. pZ wiederum ist ein Derivat des pACYC-Plasmids. pAgro4 ist beispielsweise beschrieben in Nucleic Acids Res., 1997, 25, 1203, Mol.Microbiol., 2001, 40, 397.
pHSG575ist ein Abkömmling von pSC101 mit laclq-Repressorgen aus E. coli und beispilelsweise beschrieben in Gene, 1987, 61, 63, Mol.Microbiol., 2001, 40, 397

TG10⁺(OYE) wurde von TG10⁺ durch Einführung eines weiteren Plasmids abgeleitet:
pDHE 1650 (langsamer Rhamnosepromoter + Ampicillinresistenz + ein oye-Gen).
pDHE1650 ist ein Abkömmling von pJOE2702 bei dem Gene für die ,old yellow enzymes' zwischen die Ndel und Pstl- bzw. Hindlll-Schnittstellen von pJOE2702 kloniert wurde. pJOE2702 wiederum wurde hergestellt, indem die rhaB Sequenz aus E. coli JM109 amplifiziert und in die Sphl bzw. EcoRI Schnittstellen des pBR322-Abkömmlings pBTAC1 kloniert wurde. Die Ribosomenbindestelle von pET11a sowie eine Ndel Schnittstelle wurden mit synthetischen Oligonukleotiden gewonnen und zwischen die BämHl / EcoRI Schnittstellen von pBTAC1 gesetzt. Das Plasmid ist beispielsweise beschrieben in Methods Enzymol., 1992, 216, 457, Mol.Microbiol., 1996, 21,1037.

### Auf obige Literaturstellen wird hiermit ausdrücklich Bezug genommen

Die folgenden Old Yellow Enzyme (oye) Gene wurde kloniert:
a) oye 1: Saccaromyces carlsbergensis (Genbank Q02899)
b) oye 2: Saccaromyces cerevisiae (Genbank Q03558)
c) oye 3: Saccaromyces cerevisiae (Genbank P41816).

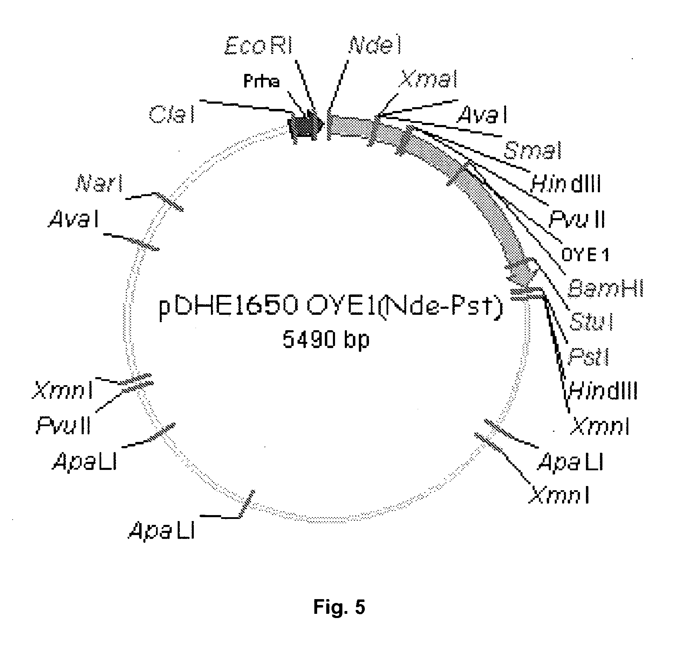

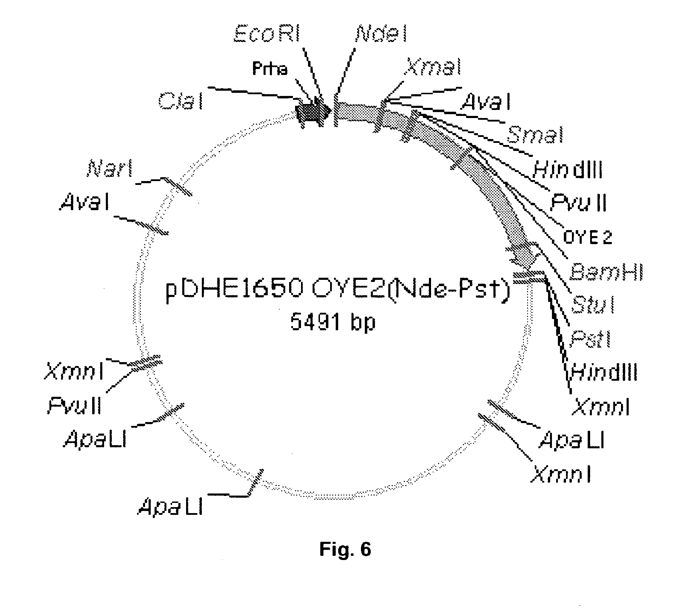

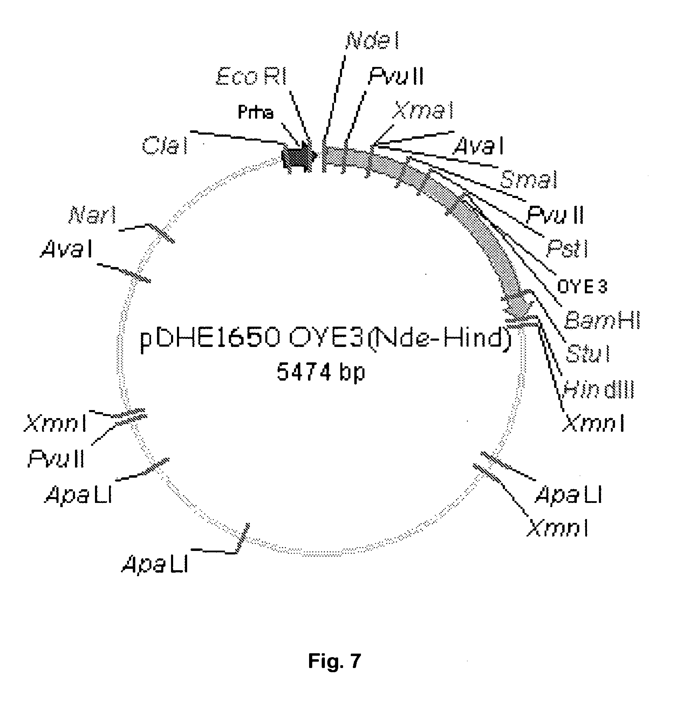

Folgende Plasmide wurden dabei erhalten:
pDHE1650 OYE1 (vgl. Figur 5)
pDHE1650 OYE2 (vgl. Figur 6)
pDHE1650 OYE3 (vgl. Figur 7)

### Beispiel 2: Biotransformationsexperimente

### a) Enzymproduktion

Die rekombinanten Stämme wurden in Luria-Broth-Medium (10 g/l Trypton, 10 g/l NaCl und 5 g/l Hefeextrakt), enthaltend 100 µg/ml Ampicillin, 100 µg/ml Streptomycin und 20 µg/ml Chloramphenicol, gezüchtet. Die Proteinüberexpression wurde mit 0,1 mM IPTG für die beiden Chaperone und 0,5 g/l L-Rhamnose für OYE induziert. Die Kulturen wurden mit 120 Upm 22 Stunden bei 37°C geschüttelt. Die geernteten Zellen wurden bei - 20°C aufgewahrt.

### a) Biotransformation

Die Biotransformationen wurden in einem Reaktionsvolumen von 1 ml unter magnetischem Rühren bei 30°C über einen Zeitraum von 1 Stunde ausgeführt. Die Anfangskonzentrationen in dem Reaktionsgemisch waren folgende: 10 g Zelltrockenmasse/l, 10 % Isopropanol [Volumen/Volumen], 20 mM 4-Phenyl-3-butin-2-on, 5 mM EDTA, 2 mM NADP⁺, 1 U/ml Glucosedehydrogenase aus Thermoplasma acidophilum, 50 mM D-Glucose, 50 mM MES-Puffer, eingestellt mit KOH auf pH 6,8. Für Biotransformationen mit NADH wurde das NADPH-Regenerationssystem (NADP⁺, Glucose dehydrogenase und Glucose) durch 15 mM NADH ersetzt.

### b) Auswertung der Versuche

Die Reaktionsgemische wurden mit Chloroform extrahiert und die Extrakte wurden durch Kapillargaschromatographie (GC) unter Verwendung eines Varian Star 3400 GC mit einer Supelco BPX5-Kapillarsäure (25 m x 0,32 mm Innendurchmesser mit einer Filmdicke der stationären Phase von 0,5 µm) und FID-Detektion nachgewiesen. Die Identität der Produkte wurde durch Co-Elution von Probenstandards und durch GC/MS-Daten belegt. Die GC/MS wurde auf einem Hewlett-Packard 5890, Serie II, Gaschromatographen, verbunden mit einem Hewlett-Packard 5972 TID Massenspektrometer unter Verwendung einer Restek RTX-5MS Kapillarsäule (30 m x 0,25 mm Innendurchmesser mit einer Filmdicke der stationären Phase von 0,25 µm) durchgeführt. Chromatogramme und die m/z-Verhältnisse wurden mit Hilfe der MSD ChemStation Software von Agilent Technologies analysiert. Zusätzlich wurden Datenbanken mit den Fragmentierungsmustern mit Hilfe der Wiley6 Library zur Identifizierung unbekannter Verbindungen durchsucht. Ein Bruker 300 MHz GYRO NMR wurde zur Bestimmung der cis- und trans-Konfiguration des gebildeten Produktes 4-phenyl-3-butene-2-one verwendet. 1D-WINNMR-Software wurde zur Datenanalyse verwendet und die erhaltenen Spektren wurden mit einem Standard von trans-4-Phenyl-3-buten-2-on mit einer Reinheit von 99 % verglichen.

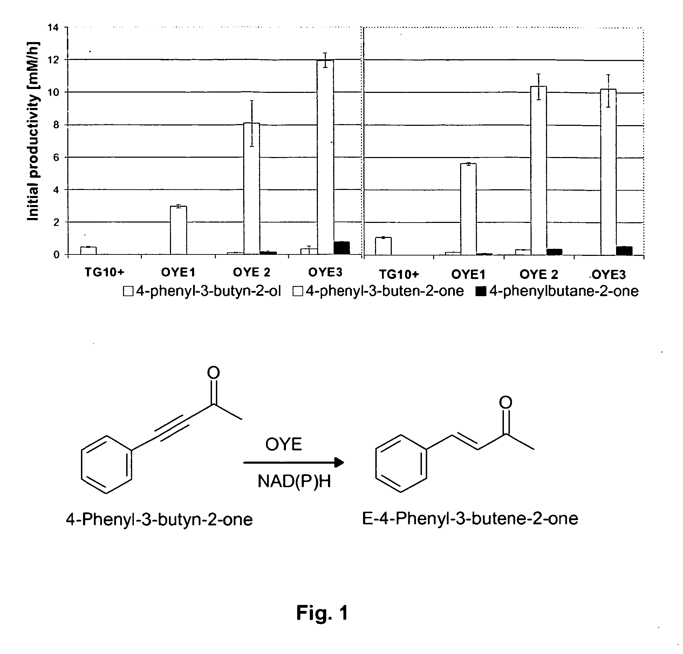

Die ermittelten Versuchsergebnisse, ausgedrückt über die anfängliche Produktivität (mM/h) sind in beiliegender Figur 1 zusammengefasst. Man beobachtet eine überraschend spezifische Bildung von E-4-Phenyl-3-buten-2-on.

## Patentansprüche

1. Verfahren zur enzymatischen Herstellung von Alkenon-Derivaten der allgemeinen Formel (2) aus α,β- ungesättigten Alkinon-Derivaten der allgemeinen Formel (1) 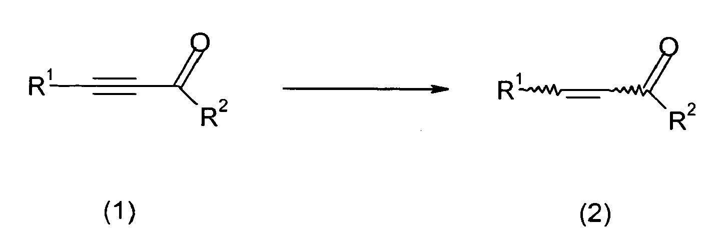 worin
R¹ für H, C₁-C₆- Alkyl, C₂-C₆- Alkenyl, oder einen gegebenenfalls substituierten carbo- oder heterocyclischen, aromatischen oder nichtaromatischen Rest steht, R² für H, C₁-C₆- Alkyl oder C₂-C₆-Alkenyl steht,
durch Reduktion einer Verbindung der Formel (1) in Gegenwart einer Reduktase
(i) umfassend wenigstens eine der Polypeptidsequenzen SEQ ID NO:1, 2, 3, 5, 7 oder 9 oder
(ii) mit einer funktional äquivalenten Polypeptidsequenz, die mindestens 80% Sequenzidentität mit SEQ ID NO:1, 2, 3, 5, 7 oder 9 aufweist.

2. Verfahren nach Anspruch 1, **dadurch gekennzeichnet, dass** die Reduktion ATPunabhängig mit NADPH oder NADH als Cofaktor durchgeführt wird.

3. Verfahren nach Anspruch 2, **dadurch gekennzeichnet, dass** der verwendete Cofaktor enzymatisch regeneriert wird.

4. Verfahren nach Anspruch 3, **dadurch gekennzeichnet, dass** die Cofaktor-Regenerierung durch Glucose-Dehydrogenase erfolgt.

5. Verfahren nach einem der vorhergehenden Ansprüche, **dadurch gekennzeichnet, dass** die Reduktion in einem wässrigen, wässrig-alkoholischen oder alkoholischen Reaktionsmedium erfolgt.

6. Verfahren nach einem der vorhergehenden Ansprüche, **dadurch gekennzeichnet, dass** die Reduktase immobilisiert vorliegt.

7. Verfahren nach einem der vorhergehenden Ansprüche, wobei das Enzym ausgewählt ist unter Reduktasen aus *Saccharomyces carlsbergensis* (Genbank Q02899), *Saccharomyces cerevisiae* (Genbank Q03558) und *Saccharomyces cerevisiae* (Genbank P41816).

8. Verfahren nach einem der vorhergehenden Ansprüche, wobei eine Verbindung der Formel (1) umgesetzt wird, worin R¹ für gegebenenfalls substituiertes Aryl steht und R² für C₁-C₆- Alkyl steht.

9. Verfahren nach einem der vorhergehenden Ansprüche, wobei das E-Isomer von Verbindungen der Formel (2) anfällt.

10. Verfahren nach einem der vorhergehenden Ansprüche, wobei die Reaktion bei einer Temperatur im Bereich von 0 bis 45°C und/oder einem pH-Wert im Bereich von 6 bis 8 erfolgt.

11. Verwendung einer Verbindung der Formel (2), hergestellt nach einem Verfahren nach einem der vorhergehenden Ansprüche als Zwischenprodukt für die chemische oder enzymatische Wirkstoffsynthese.

## Claims

1. A method for the enzymatic preparation of alkenone derivatives of the general formula (2) from α,β-unsaturated alkynone derivatives of the general formula (1) 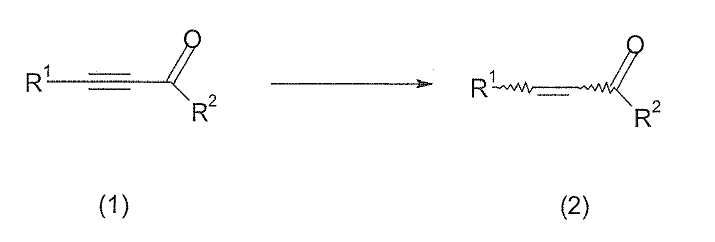 in which
R¹ is H, C₁-C₆-alkyl, C₂-C₆-alkenyl, or an optionally substituted carbo- or heterocyclic aromatic or nonaromatic ring, and
R² is H, C₁-C₆-alkyl or C₂-C₆-alkenyl,
by reducing a compound of the formula (1) in the presence of a reductase
(i) comprising at least one of the polypeptide sequences SEQ ID NO:1, 2, 3, 5, 7 or 9 or
(ii) with a functionally equivalent polypeptide sequence which has at least 80% sequence identity with SEQ ID NO:1, 2, 3, 5, 7 or 9.

2. The method according to claim 1, wherein the reduction is carried out ATP-independently with NADPH or NADH as cofactor.

3. The method according to claim 2, wherein the cofactor used is regenerated enzymatically.

4. The method according to claim 3, wherein the cofactor is regenerated by glucose dehydrogenase.

5. The method according to any of the preceding claims, wherein the reduction takes place in an aqueous, aqueous-alcoholic or alcoholic reaction medium.

6. The method according to any of the preceding claims, wherein the reductase is in immobilized form.

7. The method according to any of the preceding claims, where the enzyme is selected from reductases from *Saccharomyces carlsbergensis* (Genbank Q02899), *Saccharomyces cerevisiae* (Genbank Q03558) and *Saccharomyces cerevisiae* (Genbank P41816).

8. The method according to any of the preceding claims, where a compound of the formula (1) in which
R¹ is optionally substituted aryl, and R² is C₁-C₆-alkyl, is reacted.

9. The method according to any of the preceding claims, where the E isomer of compounds of the formula (2) results.

10. The method according to any of the preceding claims, where the reaction takes place at a temperature in the range from 0 to 45°C and/or a pH in the range from 6 to 8.

11. The use of a compound of the formula (2) prepared by a method according to any of the preceding claims as intermediate for the chemical or enzymatic synthesis of active ingredients.

## Revendications

1. Procédé pour la préparation enzymatique de dérivés d'alcénones de formule générale (2) à partir de dérivés d'alcynones α,β-insaturés de formule générale (1) 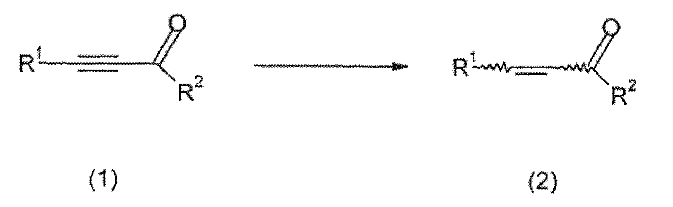 où
R¹ représente H, C₁-C₆-alkyle, C₂-C₆-alcényle, ou un radical le cas échéant substitué, carbocyclique ou hétérocyclique, aromatique ou non aromatique,
R² représente H, C₁-C₆-alkyle ou C₂-C₆-alcényle,
par réduction d'un composé de formule (1) en présence d'une réductase
(i) comprenant au moins une des séquences polypeptidiques SEQ ID NO : 1, 2, 3, 5, 7 ou 9 ou
(ii) avec une séquence polypeptidique fonctionnellement équivalente, qui présente une identité de séquence d'au moins 80% avec SEQ ID NO : 1, 2, 3, 5, 7 ou 9.

2. Procédé selon la revendication 1, **caractérisé en ce que** la réduction est réalisée indépendamment d'ATP avec du NADPH ou du NADH comme cofacteur.

3. Procédé selon la revendication 2, **caractérisé en ce que** le cofacteur utilisé est régénéré enzymatiquement.

4. Procédé selon la revendication 3, **caractérisé en ce que** la régénération du cofacteur est réalisée par la glucose-déshydrogénase.

5. Procédé selon l'une quelconque des revendications précédentes, **caractérisé en ce que** la réduction est réalisée dans un milieu réactionnel aqueux, aqueux-alcoolique ou alcoolique.

6. Procédé selon l'une quelconque des revendications précédentes, **caractérisé en ce que** la réductase se trouve sous forme immobilisée.

7. Procédé selon l'une quelconque des revendications précédentes, dans lequel l'enzyme est choisie parmi les réductases de Saccharomyces carlsbergensis (Genbank Q02899), Saccharomyces cerevisiae (Genbank Q03558) et Saccharomyces cerevisiae (Genbank P41816).

8. Procédé selon l'une quelconque des revendications précédentes, dans lequel on transforme un composé de formule (1), où
R¹ représente aryle le cas échéant substitué et R² représente C₁-C₆-alkyle.

9. Procédé selon l'une quelconque des revendications précédentes, dans lequel on obtient l'isomère E de composés de formule (2).

10. Procédé selon l'une quelconque des revendications précédentes, dans lequel la réaction est réalisée à une température dans la plage de 0 à 45°C et/ou dans une plage de pH de 6 à 8.

11. Utilisation d'un composé de formule (2), préparé selon un procédé selon l'une quelconque des revendications précédentes comme produit intermédiaire pour la synthèse de substances actives chimiques ou enzymatiques.
